# EUROPÄISCHE PATENTSCHRIFT

(11) **EP 4 072 490 B1**
(45) Veröffentlichungstag und Bekanntmachung des Hinweises auf die Patenterteilung: **12.03.2025**
(21) Anmeldenummer: 20765227.2
(22) Anmeldetag: 27.08.2020
(51) Int. Cl.: A61F 13/15, A61F 13/49, B32B 37/15

(54) **DEHNBARES WINDELELEMENT**
ELASTIC DIAPER ELEMENT
ÉLÉMENT DE COUCHE ÉLASTIQUE

(30) Priorität: 12.12.2019 DE 102019134100
(43) Veröffentlichungstag der Anmeldung: 19.10.2022
(73) Patentinhaber: RKW SE, 68309 Mannheim (DE)
(72) Erfinder: BALDAUF, Georg, 10405 Berlin (DE); WILLING, Christoph, 48691 Vreden (DE)
(74) Vertreter: Busch, Tobias
(86) Internationale Anmeldenummer: PCT/EP2020/073933
(87) Internationale Veröffentlichungsnummer: WO 2021/115642

(56) Entgegenhaltungen:
- EP-A1- 0 182 942
- DE-A1- 102010 019 702
- DE-A1- 102018 104 533
- US-A- 5 769 993

## Beschreibung

Die Erfindung betrifft ein dehnbares Windelelement mit einer elastischen Lage und einer Lage aus Nonwoven, wobei das elastische Element Verbindungsbereiche zwischen der elastische Lage und der Lage aus Nonwoven aufweist und die Verbindungsbereiche eine Erstreckung in eine Vorzugsrichtung aufweisen, wobei die Lage aus Nonwoven im ungedehnten Zustand des Windelelements gewellt vorliegt, um Bereiche als Reserve zur Ermöglichung einer Dehnung zu gewährleisten.

Bei Windeln kommen elastische Elemente zum Einsatz, um eine gute Passform und Dichtigkeit zu gewährleisten. Das erfindungsgemäße Windelelement kommt vorzugsweise als Windelbund, als sogenanntes "Waistband", zum Einsatz. Für die Verwendung als elastisches Verschlusselement, sog. "Back Ear" an Babywindeln, ist das Material ebenfalls besonders geeignet.

Das erfindungsgemäße Windelelement umfasst eine Lage aus einem elastischen Material. Die elastische Lage ist an mindestens einer Seite mit einer Lage aus einem Vliesstoff versehen. Solche Vliesstoffe weisen in der Regel eine begrenzte Dehnbarkeit auf. Bei den erfindungsgemäßen Windelbund-Elementen ist eine daher Nonwoven-Lage in einer gewellt ausgebildeten Form mit der elastischen Lage verbunden. Die Wellentäler sind mit der elastischen Lage verbunden, während die Wellenberge von der elastischen Lage wegragen und einen Hohlraum zur elastischen Lage hin einschließen. Durch die Wellen bildet die Nonwoven-Lage im ungedehnten Zustand eine Reserve zur Ermöglichung einer Dehnung des Elements.

Die DE 602 04 588 T2 beschreibt ein Herstellungsverfahren für ein streckbares elastisches Verbundmaterial. Dabei kommen Walzen zum Einsatz die eine Vielzahl von axial beabstandeten, nebeneinander liegenden, in Umfangsrichtung verlaufenden, gleich geformten Zähnen aufweisen. Die Abstände zwischen nebeneinander liegenden Zähnen bilden vertiefte, in Umfangsrichtung verlaufende, gleich konfigurierte Rillen.

In der DE 689 23 866 T2 wird eine Windel mit einer oberen und einer unteren Schicht beschrieben. Es erfolgt eine Befestigung eines elastischen Bandes an der elastischen Schicht im ungespannten Zustand. Das elastische Band wird ganzflächig mit der elastischen Schicht verbunden. Nach dem Dehnen und Entspannen des elastischen Bandes bilden sich geraffte Bereiche aus.

Die EP 217032 B1 betrifft ein Laminat mit einem elastischen Material, das an zueinander beabstandeten Stellen mit mindestens einer in Falten zu legenden Bahn verbunden ist. Bei dem elastischen Material handelt es sich um eine nicht gewellte, elastische Faserbahn.

In der EP 1 807 035 B1 wird ein Verfahren zur Herstellung eines gewellten Dehnungslaminates beschrieben. Dabei wird eine elastische Zusammensetzung in einem geschmolzenen Zustand auf eine Trägerbahn zur Bildung eines elastischen Elements aufgebracht. Es erfolgt die Bildung einer Dehnungsverbund-Vorform durch Dehnen der Trägerbahn. Dann erfolgt ein Strecken der Vorform. Ein Substrat wird mit der gestreckten Vorform verbunden, um ein gewelltes Dehnungslaminat bei einer Entspannung der gestreckten Vorform zu bilden.

Die EP 2 024 178 B1 beschreibt ein Verfahren zur Herstellung eines elastisch dehnbaren Laminats mit drei Lagen. Das Laminat umfasst eine elastische Folie und zwei Lagen aus nicht elastischem Vliesgewebe. Bei einer Variante kommt ein Kreppvlies zum Einsatz. Ein erstes elastisches Laminat wird mit einer nicht elastischen Vlieslage in einem gedehnten Zustand verbunden.

Die EP 0 182 942 A1 offenbart ein Verfahren zum Anbringen von elastischen Bändern an Bahnen zur Windeln zu formen, beinhaltet das Extrudieren eines thermoplastischen Gummis thermoplastischen Kautschuk auf die Bahn, wobei der Kautschuk mit dem durch Klebstoff gesichert ist. Ein Extruderkopf ist beweglich, um Querschnitt zu formen und nichtlineare nichtlineare Ablagerungsmuster zu erzeugen, um eine Windel mit einer bequemen Sitz zu schaffen.

Die EP 0 627 933 B1 beschreibt ein Verfahren zur Herstellung einer elastischen, mehrschichtigen Materialbahn, die aus einer flexiblen, elastischen Trägerfolie aus einem thermoplastischen Elastomer und aus je einer auf der Ober- und Unterseite der Trägerfolie wellenförmig befestigten Vliesbahn besteht, wobei letztere miteinander verschweißt sind und die Schweißverbindungen über die Materialbahn verteilt sind und gleichzeitig Bereiche erhöhter Luftdurchlässigkeit ergeben,
Die DE 10 2010 019 702 A1 offenbart ein Verfahren zur Herstellung einer elastischen Vliesstoffverbundmaterials, wobei partikelförmige Materialien auf die Vliesstofflagen aufgebracht werden, die nach dem Verbinden der Vliesstofflagen in einem Freiraum angeordnet sind.

Aufgabe der vorliegenden Erfindung ist es, ein Windelelement bereitzustellen, das günstige Dehneigenschaften aufweist und gleichzeitig eine hohe Reißfestigkeit gewährleistet. Das Element soll sich bei einer Kraftbeanspruchung dehnbar verhalten, aber auch einen ausreichenden Widerstand aufbauen, der dem Verbraucher ein Gefühl eines hochwertigen Produkts verleiht. Bei der Verwendung als "Back Ear" darf das Element nicht reißen da sich ansonsten die Windel nicht mehr verschließen lässt. Dabei soll mindestens eine Nonwoven-Lage durch eine wellige Ausbildung eine ausreichende, aber nicht zu große Reserve für eine Streckung des Elements bereitstellen. Das Laminat soll gesundheitlich unbedenklich und ökologisch nachhaltig sein. Zudem sollen keine Gerüche von dem Produkt ausgehen. Weiterhin soll das Element eine angenehme Haptik aufweisen. Zudem soll durch das Windelelement eine optimale Passform der Windel am Körper gewährleistet werden, so dass Hohlräume zwischen Windel und Körper vermieden werden und ein Auslaufen von Flüssigkeit verhindert wird.

Diese Aufgabe wird erfindungsgemäß durch ein Windelelement, ein Verfahren und eine Verwendung gemäß den nebengeordneten Hauptansprüchen gewährleistet. Bevorzugte Varianten sind den Unteransprüchen, der Beschreibung, dem Ausführungsbeispiel und der Zeichnung zu entnehmen.

Erfindungsgemäß weisen die Verbindungsbereiche in ihrer Erstreckung Unterbrechungen auf, wobei die Unterbrechungen im Verhältnis zur Gesamtfläche der Verbindungsbereiche mehr als 5 Flächenprozent und/oder weniger als 90 Flächenprozent aufweisen. Zur Herstellung des elastischen Laminats kommt vorzugsweise ein Verfahren zum Einsatz, bei dem ein Eindrücken der gewellten Nonwoven-Lage in die schmelzflüssige elastische Lage mit einer Walze erfolgt, die Erhebungen aufweist. Erfindungsgemäß wird dabei eine Walze eingesetzt, deren Erhebungen Unterbrechungen aufweist.

Vorzugsweise betragen die Unterbrechungen im Verhältnis zur Gesamtfläche der Verbindungsbereiche mehr als 5 Flächenprozent, vorzugsweise mehr als 20 Flächenprozent, insbesondere mehr als 30 Flächenprozent.

Weiterhin hat es sich als besonders günstig erweisen, wenn die Unterbrechungen im Verhältnis zur Gesamtfläche der Verbindungsbereiche weniger als 90 Flächenprozent, vorzugsweise weniger als 80 Flächenprozent, insbesondere weniger als 70 Flächenprozent aufweisen.

Als besonders günstig erweist es sich, wenn Verbindungsbereiche eine gerade Ausrichtung aufweisen. Dabei können die Verbindungsbereiche als gerade Linien ausgebildet sein.

Als besonders günstig erweist es sich, wenn die Verbindungsbereiche eine Erstreckung senkrecht zur Zugrichtung des Windelelements aufweisen.

Vorzugsweise sind die Verbindungsbereiche als unterbrochenen Linien ausgeführt. Dabei erweist es sich als günstig, wenn der Abstand dieser Linien, mehr als 1 mm vorzugsweise mehr als 2 mm, insbesondere mehr als 2,5 mm und/oder weniger als 8 mm vorzugsweise weniger als 7 mm, insbesondere weniger als 6 mm beträgt.

Bei einer vorteilhaften Variante der Erfindung wechseln sich Segmente an Verbindungsbereichen mit Segmenten an Unterbrechungen ab. Dabei werden Reihen gebildet mit unmittelbar zueinander versetzt angeordneten Segmenten von Verbindungsbereichen und Segmenten von Unterbrechungen.

Bei einer Variante der Erfindung kann das gewellte Profil der Nonwoven-Lage zwischen zwei Walzen gebildet werden, die Erhebungen und Vertiefungen aufweisen, wobei mindestens eine der beiden Walzen als Kammwalze ausgebildet ist. Die Erhebungen der einen Walze greifen dabei in die Vertiefungen der anderen Walze ein und umgekehrt. Erfindungsgemäß weisen die Erhebungen Unterbrechungen auf.

Bei einer alternativen Variante der Erfindung wird das gewellte Profil der Nonwoven-Lage zwischen einer Walze und einem weiteren Element gebildet, das wie die Walze auch Erhebungen und Vertiefungen aufweist. Die Erhebungen der Walze greifen dabei in die Vertiefungen des Elements ein und umgekehrt. Das Element erstreckt sich bogenförmig bis zur extrudierten elastischen Lage, so dass das gewellte Profil der Nonwoven-Lage bis zu dem Verbindungsschritt erhalten bleibt.

Die Ausdrücke "Nonwoven" bzw. "Vlies" beziehen sich auf einen Stoff, der aus kontinuierlichen Filamenten und/oder diskontinuierlichen Fasern ohne Weben oder Stricken mittels Verfahren wie Spunbonding, Kardieren oder Meltblowing hergestellt werden kann. Der Vliesstoff kann eine oder mehrere Lagen Vlies umfassen, wobei jede Lage kontinuierliche Filamente oder diskontinuierliche Fasern enthalten kann. Vlies kann auch Bikomponentenfasern umfassen, welche Faserstrukturen wie zum Beispiel Mantel/Kern-, Seit-an-Seit aufweisen können.

Der Ausdruck "elastisch" bezieht sich vorzugsweise auf jedes Material, das sich nach Anlegen einer gerichteten Kraft auf eine gedehnte Länge von mindestens ungefähr 160% seiner entspannten, ursprünglichen Länge strecken kann, ohne zu reißen oder zu brechen und das sich nach dem Absetzen der angelegten Kraft um mindestens ungefähr 55 % seiner Verlängerung rückstellt, vorzugsweise im Wesentlichen auf seine ursprüngliche Länge, d.h. die rückgestellte Länge beträgt weniger als ungefähr 120%, vorzugsweise weniger als ungefähr 110%, mehr bevorzugt weniger als ungefähr 105% der entspannten ursprünglichen Länge.

Der Ausdruck "Windel" bezieht sich vorzugweise auf Einweg-Absorptionsartikel, welche Fluide absorbieren und einschließen. Der Begriff umfasst unter anderem Windeln mit Verschlüssen, Windelhöschen, Übungshöschen, Schwimmwideln, Inkontinenzartikel für Erwachsene und dergleichen.

Bei der Herstellung des Windelelements wird die Nonwovenlage vor dem Verbindungsschritt in eine dreidimensionale, gewellte Form gebracht, indem sie über eine spezielle Vorrichtung geführt wird. Bei dieser Vorrichtung kann es sich um eine Walze handeln, die Erhebungen aufweist und dadurch das Wellenprofil der Nonwovenlage ausbildet. Ergänzend oder alternativ kann die Nonwovenlage vor dem Verbindungsschritt über ein Element geführt werden, das sich bogenförmig bis zur extrudierten elastischen Lage erstreckt, so dass das gewellte Profil der Nonwovenlage bis zu dem Verbindungsschritt erhalten bleibt. Das Element kann beispielsweise als Fingerleiste ausgebildet sein.

Die Herstellung des Laminats erfordert neben der Aufschmelzleistung des Extruders keine Energie von außen, wie dies beispielsweise beim Ultraschallverschweißen der Fall wäre. Die Schmelze erstarrt und es bilden sich die erfindungsgemäßen Verbindungsbereiche. Die Verbindungsbereiche werden somit auf eine Weise geschaffen, dass die Nonwoven-Lage keine thermische Belastung von außen erfährt, wie dies zum Beispiel beim Ultraschallverschweißen oder einem Aufschmelzen der elastischen Lage mittels Heizwalzen der Fall wäre.

Die erfindungsgemäßen Verbindungsbereiche sind vorzugsweise streifenartig in nebeneinander angeordneten Reihen ausgebildet. Innerhalb einer Reihe liegt vorzugsweise ein gerader Verlauf vor. Die Ausrichtung der Verbindungsbereiche ist vorzugsweise senkrecht zur Zugrichtung des Windelelements, so dass die einzelnen Reihen quer zur Zugrichtung des Windelbundes ausgerichtet sind.

Wichtig für ein optimales Windelbundlaminat ist das Verhältnis der hervorgehobenen zu den abgesenkten Bereichen der Vorrichtungen, die zur Bildung des Wellenprofils und beim Verbindungsschritt eingesetzt werden. Dieses Verhältnis wird auch als Steg-Nut-Verhältnis bezeichnet. Vorzugsweise beträgt dieses Steg zu Nut-Verhältnis weniger als 1:1, vorzugsweise weniger als 1:2.

Als besonders günstig erweist es sich, wenn die Verbindungsbereiche eine Breite von mehr als 0,1 mm, vorzugsweise mehr als 0,3 mm, insbesondere von mehr als 0,5 mm und/oder eine Breite von weniger als 1,5 mm, vorzugsweise weniger als 1,3 mm, insbesondere weniger als 0,9 mm aufweisen.

Bei einer bevorzugten Variante der Erfindung weisen die Verbindungsbereiche außenliegende Zonen auf, in denen kein elastisches Material eingedrungen ist. In den außenliegenden Zonen der Verbindungsbereiche liegt kein formschlüssiger Verbund vor, da diese Zonen frei sind von elastischem Material. Durch die außenliegenden Zonen wird ein Durchschlagen von elastischem Material durch die Nonwoven-Lage verhindert.

In den inneren Zonen der Verbindungsbereiche liegt ein formschlüssiger Verbund von erstarrtem elastischem Material und Nonwovenmaterial vor. Dabei ist das Nonwovenmaterial auch ein den inneren Zonen vorzugsweise nicht angeschmolzen, sondern die Fasern sind lediglich in die elastische Schmelze hineingedrückt. Das Material der elastischen Lage umgibt die Filamente der Nonwoven-Lage, so dass nach der Erstarrung der elastischen Lage ein formschlüssiger Verbund in der inneren Zone der Verbindungsbereiche entsteht.

Es kann in Einzelfällen auch vorkommen, dass in der inneren Zone zumindest einzelne Faser des Nonwovenmaterials angeschmolzen sein. Auch ist rein theoretisch möglich, dass in der inneren Zone das Nonwovenmaterial vollständig geschmolzen in der elastischen Schmelze vorliegt.

In allen Fällen liegt nach einer Erstarrung des elastischen Materials in der inneren Zone ein formschlüssiger Verbund von Nonwovenmaterial und erstarrtem Material der elastischen Schicht vor.

Zwischen den Verbindungsbereichen sind Bereiche angeordnet, in denen die gewellte Nonwovenlage mit ihren Erhebungen von der elastischen Lage wellenförmig wegragt und Hohlräume einschließt. Diese Bereiche dienen als Reserve für die Dehnung des Windelelements, wobei in diesen Bereichen keine Verbindung zwischen der Nonwoven-Lage und der elastischen Lage vorliegt.

Auf die Gesamtfläche der flachen Folie bezogen weisen die Reservebereiche einen deutlich größeren Anteil als die Verbindungsbereiche auf. Vorzugsweise beträgt der Anteil der Reservebereiche mehr als 60 %, insbesondere mehr als 70 %, bevorzugt mehr als 80 % der Gesamtfläche. Als Gesamtfläche wird die Oberfläche der erstarrten flachen elastischen Lage als Bezug herangezogen.

In den Reservebereichen liegt keine Verbindung der Nonwoven-Lage mit der elastischen Lage vor.

Durch die wellige Gestaltung ist die Nonwoven-Lage eines Laminatabschnitts deutlich länger als die elastische Lage. Vorzugsweise ist die Nonwoven-Lage um mehr als den Faktor 1,5, insbesondere um mehr als den Faktor 2,0, bevorzugt um mehr als den Faktor 2,5 länger als die elastische Lage.

Bei einer bevorzugten Variante der Erfindung erfolgt nach dem Verbindungsvorgang eine Streckung des Laminats in Querrichtung. Vorzugsweise erfolgt die Dehnung unterhalb der Bruchdehnung der Nonwoven-Lage.

Als besonders günstig erweist es sich, wenn die Nichtbindungsbereiche, also die Reservebereiche, eine Breite von mehr als 1,5 mm, vorzugsweise mehr als 2 mm, insbesondere mehr als 2,5 mm und/oder eine Breite von weniger als 6 mm, vorzugsweise weniger als 5 mm, insbesondere weniger als 4 mm aufweisen.

Die Bindungsbereiche weisen vorzugsweise eine Breite von mehr 0,1 mm, insbesondere mehr als 0,2 mm, bevorzugt mehr als 0,3 mm und/oder eine Breite von weniger als 1 mm, vorzugsweise weniger als 0,8 mm, bevorzugt weniger als 0,6 mm auf.

Die Reservebereiche bzw. Verbindungsbereiche sind vorzugsweise streifenförmig quer zur Zugrichtung des Windelbunds ausgebildet.

Zur Schaffung der Verbindungsbereiche kommen vorzugsweise Walzen mit einer Oberflächenstruktur zum Einsatz, wobei die Höhe der Erhebungen mehr als 100 µm, vorzugsweise mehr als 500 µm, insbesondere mehr als 1 mm und/oder weniger als 8 mm, vorzugsweise weniger als 10 mm, insbesondere weniger als 12 mm beträgt.

Vorzugsweise besteht die Nonwoven-Schicht aus einem wasserstrahlverfestigten Vliesstoff. Durch eine Wasserstrahlvernadelung können Fasern im Vliesstoff umorientiert werden, sodass die ursprüngliche zweidimensionale Faserausrichtung in ein dreidimensionale Faserorientierung überführt wird. Die Fasern sind stärker in das Vlies eingebunden. Diese Nonwoven-Schicht weist vorzugsweise ein spezifisches Gewicht von 5 bis 80 g/m², vorzugsweise von 10 bis 70 g/m², insbesondere von 15 bis 35 g/m² auf.

Vorzugsweise handelt es sich bei der wasserstrahlverfestigten Vliesstofflage um Vliesstoffe aus Endlosfilamenten. Diese bieten aufgrund ihres Herstellungsprozesses einen Faserflor, der vorzugsweise schlaufenartig ausgebildet ist.

Als Material zur Herstellung der Endlosfilamente können spinnbare Polymere, wie beispielsweise Polyester, PLA, Polyolefine, insbesondere Polypropylen und Polyethylen zum Einsatz kommen.

Der erfindungsgemäße Einsatz von wasserstrahl-verfestigten Vliesstoffs als gewellte Nonwoven-Lage, die Reservebereiche für eine Dehnung des Windelelements bildet, ist besonders vorteilhaft. Durch die Wellenbildung verformt sich der wasserstrahl-verfestigte Vliesstoff derart, dass die Fasern in den Verbindungsbereichen gestreckt werden und dadurch vorzugsweise eine Orientierung erfahren. Dadurch werden besonders vorteilhafte Verbindungszonen geschaffen, bei denen das schmelzflüssige Material die gedehnten und ausgerichteten Filamente des wasserstrahl-verfestigten Vliesstoffs umschließt und nach der Erstarrung ein besonders günstiger formschlüssiger Verbund entsteht. Erstaunlicherweise wurde festgestellt, dass beim Einsatz eines wasserstrahl-verfestigten Vliesstoffs ein Windelelement mit besonders günstigen Eigenschaften geschaffen wird. Die Nonwoven-Lage aus dem wasserstrahl-verfestigten Vliesstoffs lässt sich besonders gut verformen. Die zugeführte Nonwovenbahn verliert beim Herstellungsprozess trotz der Wellenbildung so gut wie keine Breite.

Bei der elastischen Lage handelt es sich vorzugsweise um ein Polypropylen- - und/oder einem Polyethylen-Block-Copolymer. Die elastische Folie weist vorzugsweise ein spezifisches Gewicht von 5 bis 140 g/m², insbesondere von 10 bis 130 g/m², bevorzugt von 20 bis 40 g/m² auf.

Alternativ kann die elastische Lage auch aus einem SBC (Styrol-Block-Copolymer) oder einem elastischen Polyurethan bestehen.

Bei einer Variante der Erfindung ist die elastische Lage mehrschichtig aufgebaut und vorzugsweise als Coexfolie ausgeführt. Diese umfasst bei einer vorteilhaften Variante eine Kernschicht, "Core-Layer" und eine im Vergleich dazu deutlich dünnere "Skin-Layer". Die Skin-Layer weist vorzugsweise ein spezifisches Gewicht weniger als 5 g/m², insbesondere weniger als 4 g/m², bevorzugt weniger als von 3 g/m² auf und/oder mehr als 0,3 g/m², insbesondere mehr als 0,6 g/m², bevorzugt mehr als von 0,9 g/m².

Bei einer Variante ist die Kernschicht zwischen zwei Skin-Layer-Außenschichten eingebettet.

Die Kernschicht besteht vorzugsweise aus einem elastischen Polyolefin oder einem SBC (Styrol-Block-Colymer) oder einem Polyurethan.

Die Skin-Layer besteht vorzugsweise aus einem Polyethylen, einem Polypropylen oder einem EVA (Ethylen-Vinylacetat-Copolymer).

Bei einer günstigen Variante der Erfindung werden gefüllte Polyolefine als Skin-Layer eingesetzt. Als Füllstoffe kommen beispielsweise mineralische Materialien wie Calciumcarbonat oder Talkum zum Einsatz. Der Füllstoffanteil beträgt vorzugsweise mehr als 60 Gew.-%, insbesondere mehr als 70 Gew.-%, bevorzugt mehr als 80 Gew.-%.

Zur Bildung der Skin-Layer können auch Blends zum Einsatz kommen. Dabei eignen sich beispielsweise Blends von Polyolefinen mit Polystyrol und/oder Blends von Polyolefinen mit PLA. Bei solchen Blends ist kein Füllstoff erforderlich.

Die Skin-Lage ist so gestaltet, dass sie eine Entblockung von der klebrigen Kernschicht gewährleistet. Zudem ist die Skin-Lage leicht verformbar und lässt sich gut Dehnen.

Bei einer Variante der Erfindung umfasst das Windelelement eine Nonwoven-Schicht aus einem kardierten Vliesstoff. Der eingesetzte kardierte Vliesstoff besteht vorzugsweise aus Polypropylenfasern und/oder aus Mischungen verschiedener Fasertypen, wie zum Beispiel aus Polypropylen/Viskose, Polypropylen/Polyamid, Polypropylen/Polyester usw. Der kardierte Vliesstoff kann auch aus Polypropylen und/oder Polyethylen Copolymer bestehen. Vorzugsweise beträgt das spezifische Flächengewicht des kardierten Vliesstoffs 10 bis 40 g/m², insbesondere zwischen 15 bis 25 g/m². Der kardierte Vliesstoff kann beispielsweise mittels eines Kalanders und/oder mittels Lufteinwirkung und/oder Wasserstrahl verfestigt sein.

Bei dem Windelelement kann die elastische Lage auch zwischen zwei Lagen aus Nonwoven eingebettet sein. Dabei besteht vorzugsweise mindestens eine Lage aus einem wasserstrahl-verfestigten Vliesmaterial. Die zweite Nonwovenlage kann entweder auch aus einem wasserstrahl-verfestigten Vliesstoff oder einem kardierten Vliesstoff oder einem Spinnvliesstoff bestehen. Die zweite Nonwoven-Lage kann entweder auch gewellt ausgebildet sein oder ein flaches Profil aufweisen.

Durch die erfindungsgemäße Gestaltung des Windelelements liegt die Windel optimal am Körper an und gewährleistet eine optimale Passform. Durch die Höhe seines Faltenwurfs weist das Windelelement eine voluminöse Gestaltung auf und füllt Hohlräume zwischen Windel und Körper aus, so dass eine Leckage wirksam verhindert wird.

Das erfindungsgemäße Laminat weist vorzugsweise eine gerade Fältelung der Vliesstoffoberfläche auf und besitzt eine gute Rückstellelastizität. Die Querelastizität und plastische Verformung nach Dehnungsbeaufschlagung, auch als "Set" bezeichnet, von in Längsrichtung mit Stegen verpressten Laminaten zeigt eine proportionale Abhängigkeit mit der Verbundfläche. Bei dem erfindungsgemäßen Laminat ist die Verbundfläche verkleinert. Dabei wird gleichzeitig eine ausreichende Verbundhaftung beibehalten. Vorzugsweise beträgt die Verbundhaftung des erfindungsgemäßen Laminats mehr als 1N/25 mm in Maschinenrichtung (MD) bei gleichzeitiger Verbesserung der Elastizität In Querrichtung (CD)

Beide Anforderungen werden mit der Verringerung der Stegpressfläche der eingesetzten Walzen erreicht. Bei einer Variante der Erfindung werden die Stege der Walzen mit Einfräsungen versehen, also durchbrochen.

Vorzugsweise kommen dabei Walzen mit parallelen Stegen und Rillen zum Einsatz. Als besonders vorteilhaft haben sich umfangsidentische, wellig gerillte Walzen mit Stegen und Rillen erwiesen. Diese weisen vorzugsweise bei einer Frequenz von ca. 30 bis 100 mm eine Amplitude von ca. 1 bis 6 mm. Die mit einer Tiefe von ca. 0,5 bis 10 mm wellig gerillten Walzen greifen ineinander und rollen gegenläufig mit identischen Umfang und Geschwindigkeit ineinandergreifen ab.

Als besonders günstiger erweist sich der Einsatz von Walzen mit parallelen oder gewellten Stegen, wobei die Stege in Umlaufrichtung ein intermittierendes Höhenprofil aufweisen.

Weitere Vorteile und Merkmale der Erfindung ergeben sich aus der Beschreibung eines Ausführungsbeispiels anhand von Zeichnungen und aus den Zeichnungen selbst.

Dabei zeigt
Fig. 1 zeigt einen Schnitt durch ein erfindungsgemäßes Windelelement,
Fig. 2 eine schematische Draufsicht auf das Windelelement mit einer ersten Variante der Anordnung von Unterbrechungen.

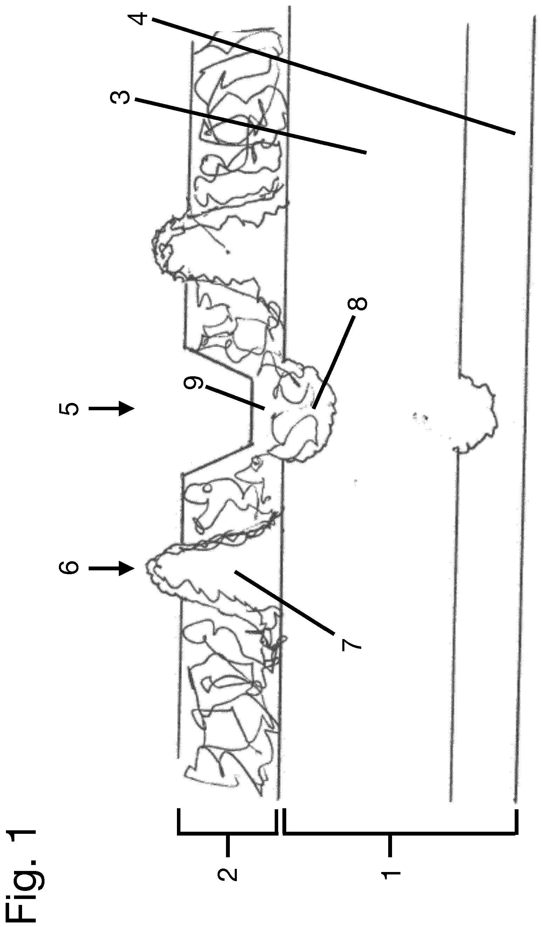

Gemäß Figur 1 umfasst das Laminat eine elastische Lage 1 und eine Nonwoven-Lage 2 aus einem gewellten Vliesstoff. Dabei handelt es sich um einen wasserstrahlverfestigten Vliesstoff, wobei im Ausführungsbeispiel ein Spinnvlies aus Endlosfilamenten zum Einsatz kommt. Erfindungsgemäß wird der Vliesstoff vor der Verbindung mit der elastische Lage 1 in eine Wellenform gebracht. Nach einem Extrudieren der elastischen Lage 1 zu der gewellten Nonwoven-Lage 2 werden die Vertiefungen des Vliesstoffes in die schmelzflüssige elastische Lage 1 gedrückt, so dass sich Verbindungsbereiche 5 zwischen dem gewellten Vliesstoff 2 und der elastischen Lage 1 ausbilden.

Im Ausführungsbeispiel ist die elastische Lage 1 als mehrschichtige Coexfolie aufgeführt, mit einer Kernschicht 3 und einer weiteren Schicht 4, die als "Skin-Layer" ausgebildet ist. Das Verhältnis der Dicke der Kernschicht 3 zur weiteren Schicht 4 beträgt vorzugsweise mehr als 8:1 insbesondere mehr als 10:1 insbesondere mehr als 12:1: Die Skin-Layer 4 weist vorzugsweise ein Gewicht zwischen 1 bis 3 g/m² auf.

Vorzugsweise besteht die Kernschicht 3 aus thermoplastischen Polymeren. Dabei kommen vorzugsweise Polypropylen-Polyethylen-Block-Copolymere zum Einsatz, beispielsweise der Typenreihe Exxon Vistamaxx (PP basiert): VM 6102, oder VM6202 oder VM 7810 und/oder der Typenreihe Dow Infuse (PE basiert): Infuse 9507, Infuse 9107.

Die Außenschicht 4 besteht vorzugsweise aus einem Polyolefin oder einem Ethylen-Vinylacetat-Copolymer (EVA). Die Außenschicht 4 ist im Gegensatz zur Kernschicht 3 nicht "klebrig" und verhindert somit ein unerwünschtes Anhaften.

Erfindungsgemäß umfasst das Laminat Verbindungsbereiche 5 und Reservebereiche 6. Die Reservebereiche 6 weisen keine oder nur eine sehr schwache Bindung mit der elastischen Lage 1 aus und schließen vorzugsweise Hohlräume 7 ein.

Im Ausführungsbeispiel beträgt das Steg zu Nut-Verhältnis der Walze, welche das Nonwoven in eine Wellenform bringt und die Vertiefungen der gewellten Nonwoven-Lage 2 in die schmelzflüssige elastische Lage 1 drückt bei 1:6, vorbei die Stegbreite vorzugsweise 0,5 mm und die Nutbreite vorzugsweise 3 mm beträgt.

Die erfindungsgemäßen Verbindungsbereiche 5 weisen im Ausführungsbeispiel unterschiedliche Zonen 8, 9 auf.

Die außenliegende Zone 9 ist frei von elastischem Material, so dass kein Durchschlagen des elastischen Materials durch die Nonwoven-Lage 2 erfolgt. Das Nonwovenmaterial in der außenliegenden Zone 8 von außen thermisch unbeeinflusst, so dass die Filamente der Lage 2 aus Nonwoven nicht angeschmolzen sind.

In der inneren Zone 8 der Verbindungsbereiche 5 liegt ein formschlüssiger Verbund von erstarrtem elastischem Material und Nonwovenmaterial vor. Dabei ist im Ausführungsbeispiel auch in der inneren Zone 8 das Nonwovenmaterial nicht angeschmolzen. Die Endlosfilamente des wasserstrahl-verfestigten Vliesstoffs sind lediglich in die elastische Schmelze hineingedrückt, so dass nach der Erstarrung ein formschlüssiger Verbund entsteht. Dabei bleiben beim Verbindungsprozess die Endlosfilamente des wasserstrahl-verfestigten Vliesstoffs selbst weitgehend unbeeinflusst. Sie werden lediglich von dem schmelzflüssigen Material der elastische Lage 1 umschlossen.

Nach einer Erstarrung des elastischen Materials liegt in der inneren Zone 8 ein formschlüssiger Verbund von Nonwovenmaterial und erstarrtem Material der elastischen Schicht 1 vor.

Das in der Figur dargestellte Laminat wird zwischen einem Walzenpaar miteinander verbunden, bei der mit Blick auf die Zeichnung von oben eine profilierte Walze mit Erhebungen die Nonwoven-Lage 2 in die elastische Lage 1 drückt und von unten eine Gegenwalze mit einer glatten Oberfläche angeordnet ist. Bei der Herstellung des in der Figur dargestellten Laminats kommt eine Kühlwalze als Gegenwalze zum Einsatz. Bei der Kühlwalze handelt es sich um eine Stahlwalze. Bei der von oben wirkenden Walze handelt es sich um eine nicht gekühlte Walze.

Die zur Verbindung eingesetzten Walzen werden auf Abstand gefahren, wobei ein fester Abstand eingestellt wird.

Das Laminat hat vorzugsweise ein spezifisches Flächengewicht von mehr als 10 g/m², insbesondere mehr als 20 g/m², bevorzugt mehr als 30 g/m² und/oder weniger als 200 g/m², insbesondere weniger als 150 g/m², bevorzugt weniger als 100 g/m².

Im Ausführungsbespiel sind die Verbindungsbereiche 5 und die Reservebereiche 6 streifenförmig ausgeführt, wobei die Streifen quer zur Zugrichtung des Windelelements verlaufen.

Die Streifen der Verbindungsbereiche 5 haben ein Breite zwischen 0,1 und 1 mm. Im Ausführungsbeispiel haben die Verbindungsbereiche 5 eine Breite von 0,5 mm.

Die Streifen der Reservebereiche 6, bei denen keine Verbindung der Nonwoven-Lage 2 mit der elastisch Lage 1 vorliegt, haben eine Breite zwischen 2 und 6 mm. Im Ausführungsbeispiel haben die Reservebereiche 6 eine Breite von 3 mm. Somit haben im Ausführungsbeispiel die Verbindungsbereiche 5 einen Anteil von 0,5/3,5 = 14,3 % und die Reservebereiche 6 einen Anteil von 3/3,5 = 85,7 % bezogen auf die Oberfläche der flachen, nicht gedehnten elastischen Lage 1.

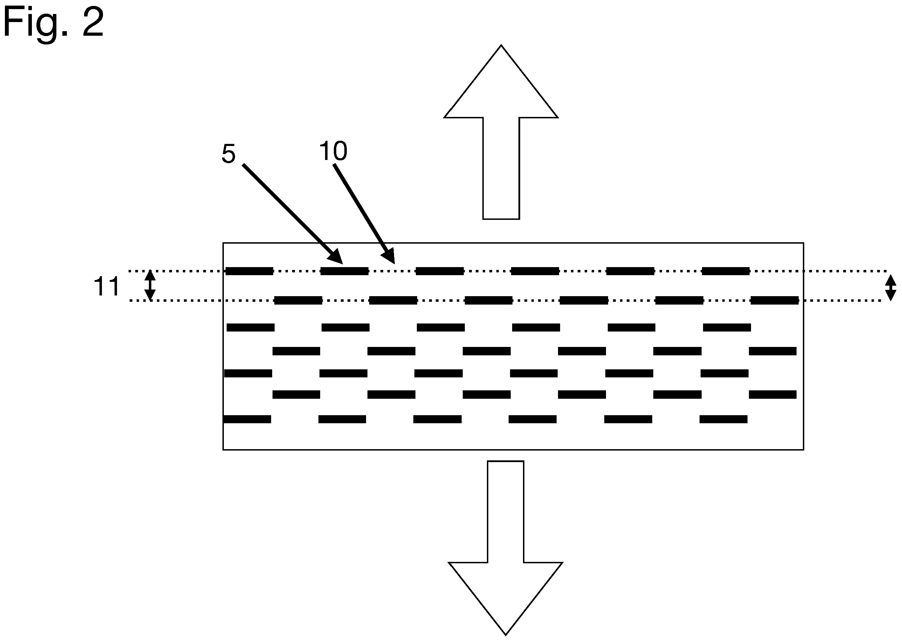

Fig. 2 zeigt eine schematische Draufsicht auf das Windelelement mit einer ersten Variante der Anordnung von Unterbrechungen. Die Verbindungsbereiche 5 erstrecken sich quer zur Zugrichtung und weisen erfindungsgemäß Unterbrechungen 10 auf. Die Verbindungsbereiche 5 sind als unterbrochenen Linien ausgeführt sind, wobei der Abstand 11 dieser Linien im Ausführungsbeispiel ca. 3 mm beträgt. Die mit Blick auf die Zeichnung nach oben und unten weisenden Blockpfeile zeigen die Zugrichtung bei Beanspruchung des Windelelements.

## Patentansprüche

1. Dehnbares Windelelement mit einer elastischen Lage (1) und einer Lage (2) aus Nonwoven, wobei das elastische Element Verbindungsbereiche (5) zwischen der elastische Lage (1) und der Lage (2) aus Nonwoven aufweist und die Verbindungsbereiche (5) eine Erstreckung in eine Vorzugsrichtung aufweisen, wobei die Lage (2) aus Nonwoven im ungedehnten Zustand des Windelelements gewellt vorliegt, um Bereiche (6) als Reserve zur Ermöglichung einer Dehnung zu gewährleisten, **dadurch gekennzeichnet, dass** die Verbindungsbereiche (5) in ihrer Erstreckung Unterbrechungen (10) aufweisen, wobei die Unterbrechungen (10) im Verhältnis zur Gesamtfläche der Verbindungsbereiche (5) mehr als 5 Flächenprozent und/oder weniger als 90 Flächenprozent aufweisen.

2. Windelelement nach Anspruch 1, **dadurch gekennzeichnet, dass** die Unterbrechungen (10) im Verhältnis zur Gesamtfläche der Verbindungsbereiche (5) mehr als 20 Flächenprozent, insbesondere mehr als 30 Flächenprozent und/oder weniger als 80 Flächenprozent, insbesondere weniger als 70 Flächenprozent aufweisen.

3. Windelelement nach Anspruch 1 oder 2, **dadurch gekennzeichnet, dass** die Verbindungsbereiche (5) eine gerade Erstreckung aufweisen.

4. Windelelement nach einem der Ansprüche 1 bis 3, **dadurch gekennzeichnet, dass** die Verbindungsbereiche (5) eine Erstreckung senkrecht zur Zugrichtung des Windelelements aufweisen.

5. Windelelement nach einem der Ansprüche 1 bis 4, **dadurch gekennzeichnet, dass** Verbindungsbereiche 5 als unterbrochenen Linien ausgeführt sind, wobei der Abstand 11 dieser Linien, mehr als 1 mm vorzugsweise mehr als 2 mm, insbesondere mehr als 2,5 mm und/oder weniger als 8 mm vorzugsweise weniger 7 mm als insbesondere weniger als 6 mm beträgt.

6. Windelelement nach einem der Ansprüche 1 bis 5, **dadurch gekennzeichnet, dass** die Lage (2) aus Nonwoven aus einem wasserstrahlverfestigten Vliesstoff besteht, vorzugsweise aus einem wasserstrahlverfestigten Vliesstoff aus Endlosfilamenten, wobei der Vliesstoff vorzugsweise ein spezifisches Gewicht von 10 bis 70 g/m², insbesondere 10 bis 40 g/m², bevorzugt 15 bis 35 g/m² aufweist.

7. Windelelement nach einem der Ansprüche 1 bis 6, **dadurch gekennzeichnet, dass** die elastische Lage (1) mehrschichtig aufgebaut ist und vorzugsweise eine Kernschicht (3) und mindestens eine weitere Schicht (4) aufweist.

8. Windelelement nach einem der Ansprüche 1 bis 7, **dadurch gekennzeichnet, dass** das Element eine weitere Lage aus Nonwoven aufweist, wobei die elastische Lage (1) zwischen den beiden Lagen aus Nonwoven angeordnet ist.

9. Verfahren zur Herstellung eines elastischen Laminats mit folgenden Schritten:
- Bildung einer gewellten Lage (2) aus Nonwoven,
- Extrusion einer elastischen Lage (1),
- Eindrücken der gewellten Nonwoven-Lage (2) in die schmelzflüssige elastische Lage (1) mit einer Walze die Erhebungen aufweist,
**dadurch gekennzeichnet, dass** die Erhebungen Unterbrechungen aufweisen.

10. Verwendung eines nach Anspruch 9 hergestellten Laminats als dehnbares Windelelement, insbesondere als Windelbund.

## Claims

1. Elastic diaper element comprising an elastic layer (1) and a layer (2) of nonwoven, wherein the elastic element has connecting regions (5) between the elastic layer (1) and the layer (2) of nonwoven (2), and the connecting regions (5) have an orientation in a preferred direction, wherein the layer (2) of nonwoven is corrugated in the unstretched state of the diaper element, in order to provide areas (6) as a reserve for enabling stretching **characterized in that** the connecting regions (5) have interruptions (10) in their orientation, the interruptions (10) being interruptions (10) in relation to the total area of the connecting regions (5) of more than 5 percent and/or of less than 90 percent by area.

2. Diaper element according to claim 1, **characterized in that** the interruptions (10) in relation to the total area of the connecting regions (5) have more than 20 percent by area, in particular more than 30 percent by area and/or less than 80 percent by area, in particular less than 70 percent by area.

3. Diaper element according to claim 1 or 2, **characterized in that** the connecting regions (5) have a straight orientation.

4. Diaper element according to one of claims 1 to 3, **characterized in that** the connecting regions (5) have an orientation perpendicular to the pulling direction of the diaper element.

5. Diaper element according to one of claims 1 to 4, **characterized in that** connecting regions 5 are designed as interrupted lines wherein the distance 11 between these lines is more than 1 mm preferably more than 2 mm, in particular more than 2.5 mm and/or less than 8 mm, preferably less than 7 mm, in particular less than 6 mm.

6. Diaper element according to one of claims 1 to 5, **characterized in that** the layer (2) of nonwoven consists of a of a hydroentangled nonwoven, preferably of a hydroentangled nonwoven made of continuous filaments, the nonwoven preferably having a specific weight of 10 to 70 g/m², in particular 10 to 40 g/m², preferably 15 to 35 g/m².

7. Diaper element according to one of claims 1 to 6, **characterized in that** the elastic layer (1) has a multilayer structure and preferably has a core layer (3) and at least one further layer (4).

8. Diaper element according to one of claims 1 to 7, **characterized in that** the element has a further layer of nonwoven, the elastic layer (1) being arranged between the two layers of nonwoven.

9. Method for producing an elastic laminate comprising the following steps:
- Formation of a corrugated layer (2) of nonwoven,
- extrusion of an elastic layer (1),
- pressing the corrugated nonwoven layer (2) into the molten elastic layer (1) with a roller which has elevations,
**characterized in that** the elevations have interruptions.

10. Use of a laminate produced according to claim 9 as a stretchable diaper element, in particular as a diaper waistband.

## Revendications

1. Elément de couche élastique comprenant une couche élastique (1) et une couche (2) de non-tissé, dans lequel l'élément élastique a des régions de liaison (5) entre la couche élastique (1) et la couche (2) de non-tissé (2), et les régions de liaison (5) ont une extension dans une direction préférée, dans laquelle la couche (2) de non-tissé est ondulée à l'état non étiré de l'élément de couche, afin de fournir des zones (6) comme réserve pour permettre l'étirement, **caractérisées par le fait que** les régions de liaison (5) ont des interruptions (10) dans leur extension, les interruptions (10) étant des interruptions (10) par rapport à la surface totale des régions de liaison (5) de plus de 5 pour cent et/ou de moins de 90 pour cent en surface.

2. Elément de couche selon la revendication 1, **caractérisé par le fait que** les interruptions (10) par rapport à la surface totale des régions de liaison (5) ont une surface supérieure à 20 pour cent, en particulier supérieure à 30 pour cent et/ou inférieure à 80 pour cent, en particulier inférieure à 70 pour cent.

3. Elément de couche selon la revendication 1 ou 2, **caractérisé par le fait que** les régions de liaison (5) ont une extension droite

4. Elément de couche selon l'une des revendications 1 à 3, **caractérisé par le fait que** les régions de liaison (5) ont une extension perpendiculaire à la direction de traction de l'élément de couche.

5. Elément de couche selon l'une des revendications 1 à 4, **caractérisé par le fait que** les régions de liaison 5 sont conçues comme des lignes interrompues dans lesquelles la distance 11 entre ces lignes est supérieure à 1 mm, de préférence supérieure à 2 mm, en particulier supérieure à 2,5 mm et/ou inférieure à 8 mm, de préférence inférieure à 7 mm, en particulier inférieure à 6 mm.

6. Elément de couche selon l'une des revendications 1 à 5, **caractérisé en ce que** la couche (2) de non-tissé est constituée d'un non-tissé hydro-élaboré, de préférence d'un non-tissé hydro-élaboré en filaments continus, le non-tissé ayant de préférence un poids spécifique de 10 à 70 g/m², en particulier de 10 à 40 g/m², de préférence de 15 à 35 g/m².

7. Elément de couche selon l'une des revendications 1 à 6, **caractérisé par le fait que** la couche élastique (1) a une structure multicouche et comporte de préférence une couche centrale (3) et au moins une autre couche (4).

8. Elément de couche selon l'une des revendications 1 à 7, **caractérisé par le fait que** l'élément comporte une autre couche de non-tissé, la couche élastique (1) étant disposée entre les deux couches de non-tissé.

9. Procédé de fabrication d'un laminé élastique comprenant les étapes suivantes :
- Formation d'une couche ondulée (2) de non-tissé,
- extrusion d'une couche élastique (1),
- presser la couche non tissée ondulée (2) dans la couche élastique fondue (1) avec un rouleau qui comporte des élévations,
**caractérisé par** des interruptions dans les élévations.

10. Utilisation d'un laminé produit selon la revendication 9 comme élément de couche extensible, en particulier comme ceinture de couche.
